# EUROPEAN PATENT APPLICATION

(11) **EP 2 308 849 A1**
(43) Date of publication of application: **13.04.2011**
(21) Application number: 09382198.1
(22) Date of filing: 06.10.2009
(51) Int. Cl.: C07D 231/12, C07D 231/56, A61K 31/416, A61P 25/00

(54) **Indane-amine derivatives, their preparation and use as medicaments**

(71) Applicant: Laboratorios del. Dr. Esteve, S.A., 08041 Barcelona (ES)
(72) Inventor: Garcia-Lopez, Monica, Barcelona (ES); Torrens-Jover, Antoni, Terrassa (Barcelona) (ES)
(74) Representative: Carpintero Lopez, Francisco

(57) **Abstract**

The present invention relates to indane-amine derivatives, to processes for the preparation thereof, to medicaments comprising them as well as to their use for the preparation of a medicament for the treatment of 5HT7 receptor mediated diseases or conditions.

## Description

### FIELD OF THE INVENTION

The present invention relates to indane-amine derivatives, to processes for the preparation thereof, to medicaments comprising them as well as to their use for the preparation of a medicament for the treatment of 5HT7 receptor mediated diseases or conditions.

### BACKGROUND OF THE INVENTION

The search for new therapeutic agents has been greatly aided in recent years by better understanding of the structure of proteins and other biomolecules associated with target diseases. One important class of proteins that has been the subject of extensive study is the family of 5-hydroxytryptamine (serotonin, 5-HT) receptors. The 5-HT7 receptor discovered in 1993 belongs to this family and has attracted great interest as a valuable new drug target (Terrón, J.A. Idrugs, 1998, vol. 1, no. 3, pages 302-310: "The 5HT7 receptor: A target for novel therapeutic avenues? ").

5-HT7 receptors have been cloned from rat, mouse, guinea pig and human cDNA and exhibit a high degree of interspecies homology (approx. 95%), but it is unique in that it has a low sequence homology with other 5-HT receptors (less than 40%). Its expression pattern, in particular structures of the central nervous system (CNS) (highest in hypothalamus, in particular suprachiasmatic nuclei, and thalamus) and other peripheral tissues (spleen, kidney, intestinal, heart and coronary arthery), implicates the 5-HT7 receptor in a variety of functions and pathologies. This idea is reinforced by the fact that several therapeutic agents, such as tricyclic antidepressants, typical and atypical antipsychotics and some 5-HT2 receptor antagonists, display moderate to high affinity for both recombinant and functional 5-HT7 receptors.

Functionally, the 5-HT7 receptor has been implicated in regulation of circadian rhythms in mammals (Lovenberg, T.W. et al. Neuron, 1993, 11:449-458 "A novel adenylyl cyclase-activating serotonin receptor (5-HT7) implicated in the regulation of circadian rhythms"). It is known that disruption of circadian rhythms is related to a number of CNS disorders including depression, seasonal affective disorder, sleep disorders, shift worker syndrome and jet lag among others.

Distribution and early pharmacological data also suggest that the 5-HT7 receptor is involved in the vasodilatation of blood vessels. This has been demonstrated in vivo (Terrón, J.A., Br J Pharmacol, 1997, 121:563-571 "Role of 5-HT7 receptors in the long lasting hypotensive response induced by 5-hydroxytryptamine in the rat"). Thus, selective 5-HT7 receptor agonists have a potential as novel hypertensive agents.

The 5-HT7 receptor has also been related with the pathophysiology of migraine through smooth muscle relaxation of cerebral vessels (Schoeffter, P. et al., 1996, Br J Pharmacol, 117:993-994 ; Terrón, J.A., 2002, Eur. J. Pharmacol., 439:1-11 "Is the 5-HT7 receptor involved in the pathogenesis and prophylactic treatment of migraine? "). In a similar manner, involvement of 5-HT7 in intestinal and colon tissue smooth muscle relaxation makes this receptor a target for the treatment of irritable bowel syndrome (De Ponti, F. et al. , 2001, Drugs, 61:317-332 "Irritable bowel syndrome. New agents targeting serotonin receptor subtypes "). Recently, it has also been related to urinary incontinence ( British J. of Pharmacology, Sept. 2003, 140(1) 53-60: "Evidence for the involvement of central 5HT-7 receptors in the micurition reflex in anaeshetized female rats").

In view of the potential therapeutic applications of agonists or antagonists of the 5HT7 receptor, a great effort has been directed to find selective ligands. Despite intense research efforts in this area, very few compounds with selective 5-HT7 antagonist activity have been reported ( Wesolowska, A., Polish J. Pharmacol., 2002, 54: 327-341, "In the search for selective ligands of 5-HT5, 5-HT6 and 5-HT7 serotonin receptors "), yet even fewer 5-HT7-Agonists.

Some indane-amine derivatives showing activity towards 5HT7 receptors were described in our application EP2011786.

However, there is still a need to find compounds that have pharmacological activity towards the receptor 5-HT7, being both effective and selective, and having good "drugability" properties, i.e. good pharmaceutical properties related to administration, distribution, metabolism and excretion.

The authors of the present invention hereby provide some novel indane-amine derivatives complying with the above mentioned characteristics.

### DETAILED DESCRIPTION OF THE INVENTION

A first object of the invention refers to compounds of general formula I: 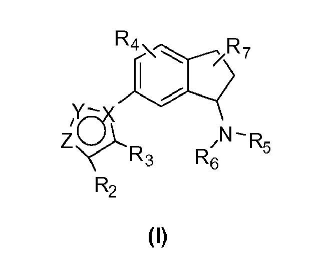 wherein X-Y-Z together form

-N-N=CR₁- or =C-NR₁-N= ;

wherein
R₁ is selected from the group consisting of hydrogen, a linear or branched, saturated or unsaturated, optionally at least mono-substituted aliphatic radical or a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group;
R₂ and R₃ are independently from each other selected from the group consisting of hydrogen; a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group; or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH
or R₂ and R₃ form an saturated or unsaturated, optionally at least mono-substituted 5- or 6-membered-heterocyclic ring, which may be condensed with an optionally at least mono-substituted mono- or polycyclic ring system;
R₄ is selected from hydrogen, halogen, OH, SH, NH_{2,} a linear or branched, saturated or unsaturated, optionally at least mono-substituted aliphatic radical or O-R with R being a linear or branched, saturated or unsaturated, optionally at least mono-substituted aliphatic radical;
R₅ and R₆ each are independently selected from the group consisting of hydrogen, a linear or branched, saturated or unsaturated, optionally at least mono-substituted aliphatic radical or
R₅ and R₆ together with the bridging nitrogen atom form a saturated or unsaturated, optionally at least mono-substituted 5- or 6-membered-heterocyclic ring, which may be condensed with an optionally at least monosubstituted mono- or polycyclic ring system;
R₇ is a hydrogen
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds of general formula I show a high affinity to the 5HT7 receptor as well as a high selectivity for this receptor in comparison to e.g. the 5HT6, the sigma 1, the sigma 2, and the 5HT1 receptor. In addition some of these compounds show an agonistic activity on this receptor.

A "mono- or polycyclic ring-system" according to the present invention means a mono-or polycyclic hydrocarbon ring-system that may be saturated, unsaturated or aromatic. If the ring-system is polycyclic, each of its different rings may show a different degree of saturation, i.e. it may be saturated, unsaturated or aromatic. Optionally each of the rings of the mono- or polycyclic ring-system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-system are preferably 5- or 6-membered.

An "aryl", "aryl radical" or group is understood as meaning ring-systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention "cycloalkyl radical" or group is understood as meaning saturated and unsaturated (but not aromatic) cyclic hydrocarbons (without a heteroatom in the ring), which can be unsubstituted or mono- or polysubstituted. Furthermore, C3-4-cycloalkyl represents C3- or C4-cycloalkyl, C3-5-cycloalkyl represents C3-, C4- or C5-cycloalkyl, C3-6-cycloalkyl represents C3-, C4-, C5- or C6-cycloalkyl, C3-7-cycloalkyl represents C3-, C4-, C5-, C6- or C7-cycloalkyl, C3-8-cycloalkyl represents C3-, C4-, C5-, C6-, C7- or C8-cycloalkyl, C4-5-cycloalkyl represents C4- or C5-cycloalkyl, C4-6-cycloalkyl represents C4-, C5- or C6-cycloalkyl, C4-7-cycloalkyl represents C4-, C5-, C6-or C7-cycloalkyl, C4-8-cycloalkyl represents C4-, C5-, C6- C7- or C8-cycloalkyl C5-6-cycloalkyl represents C5- or C6-cycloalkyl and C5-7-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The cycloalkyl radicals are preferably cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

A "heterocyclyl", a "heterocyclyl radical" or group or "heterocyclic ring-system" is understood as meaning heterocyclic ring-systems which contain one or more heteroatoms from the group consisting of nitrogen, oxygen and/or sulfur in the ring or ring-system, and can also be mono- or polysubstituted. The ring-system may consist either of only one saturated or unsaturated or even aromatic ring or may consist of 2, 3 or 4 saturated or unsaturated or even aromatic rings, which are condensed in that between two or more of the rings ring members are shared. Examples which may be mentioned from the group of heterocyclyls are furan, benzofuran, thiophene, benzothiophene, pyrrole, pyridine, pyrimidine, pyrazine, quinoline, isoquinoline, phthalazine, benzo-1,2,5-thiadiazole, imidazo-thiazole, benzothiazole, indole, benzotriazole, benzodioxolane, benzodioxane, carbazole and quinazoline.

In connection with mono- or polycyclic ring-system, aryl radical, cycloalkyl radical, or heterocyclyl radical, "substituted" is understood - unless defined otherwise - as meaning replacement of at least one hydrogen radical on the ring-system of the mono-or polycyclic ring-system, the aryl radical, the cycloalkyl radical, or the heterocyclyl radical by OH, SH, =O, halogen (F, Cl, Br, I), CN, NO₂, COOH; NRxRy, with Rx and Ry independently being either H or a saturated or unsaturated, linear or branched, substituted or unsubstituted C1-6-alkyl; by a saturated or unsaturated, linear or branched, substituted or unsubstituted C1-6-alkyl; a saturated or unsaturated, linear or branched, substituted or unsubstituted -O-C1-6-alkyl (alkoxy); a saturated or unsaturated, linear or branched, substituted or unsubstituted -S-C1-6alkyl; a saturated or unsaturated, linear or branched, substituted or unsubstituted - C(O)-C1-6-alkyl; a saturated or unsaturated, linear or branched, substituted or unsubstituted -C(O)-O-C1-6-alkyl; a substituted or unsubstituted phenyl. Within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents. Therefore, "optionally at least monsubstituted" means either "not substituted" if the option is not fulfilled, "monosubstituted" or "polysubstituted".

In connection with aryl radical, cycloalkyl radical, or heterocyclyl radical, "condensed with" is understood as meaning that the ring-system of the aryl radical, the cycloalkyl radical, or the heterocyclyl radical is sharing two atoms (one) of its ring(s) with a ring of the mono- or polycyclic ring-system it is condensed with.

Aliphatic radicals/groups, as referred to in the present invention, are optionally mono-or polysubstituted and may be branched or linear, saturated or unsaturated. Aliphatic radicals, as defined in the present invention, include alkyl, alkenyl and alkinyl radicals. Unsaturated aliphatic radicals, as defined in the present invention, include alkenyl and alkinyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl.

In the context of this invention, "alkyl", "alkyl radical" or group is understood as meaning saturated, linear or branched hydrocarbons, which can be unsubstituted or mono- or polysubstituted. Thus unsaturated alkyl is understood to encompass alkenyl and alkinyl groups, like e.g. -CH=CH-CH₃ or -C C-CH₃, while saturated alkyl encompasses e.g. - CH₃ and -CH₂-CH₃. In these radicals, C1-2-alkyl represents C1-or C2-alkyl, C1-3-alkyl represents C1-, C2- or C3-alkyl, C1-4-alkyl represents C1-, C2-, C3- or C4-alkyl, C1-5-alkyl represents C1-, C2-, C3-, C4-, or C5-alkyl, C1-6-alkyl represents C1-, C2-, C3-, C4-, C5- or C6-alkyl, C1-7alkyl represents C1-, C2-, C3-, C4-, C5-, C6- or C7-alkyl, C1-8-alkyl represents C1-, C2-, C3-, C4-, C5-, C6-, C7- or C8-alkyl, C1-10-alkyl represents C1-, C2-, C3-, C4-, C5-, C6-, C7-, C8-, C9-or C10-alkyl and C1-18-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF3 or CH₂OH etc.

In connection with alkylene, alkyl or aliphatic radical or group - unless defined otherwise - the term "substituted" in the context of this invention is understood as meaning replacement of at least one hydrogen radical by F, Cl, Br, I, NH2, SH or OH; within that "monosubstituted" means the substitution of exactly one hydrogen radical, whereas "polysubstituted" means the substitution of more than one hydrogen radical with "polysubstituted" radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. Therefore, "optionally at least monsubstituted" means either "not substituted" if the option is not fulfilled, "monosubstituted" or "polysubstituted".

The term "alkylene" is understood as meaning a divalent alkyl group like -CH₂- or - CH₂-CH₂-, with (CH₂)3-6 being understood as meaning -CH₂-CH₂-CH₂-, -CH₂-CH₂-CH₂-CH₂, -CH₂-CH₂-CH₂-CH₂-CH₂- and -CH₂-CH₂-CH₂-CH₂-CH₂-CH₂-, (CH₂)1-4 is to be understood as meaning -CH₂-, -CH₂-CH₂-, -CH₂-CH₂-CH₂- and -CH₂-CH₂-CH₂-CH₂-, (CH₂)4-5 is to be understood as meaning -CH₂-CH₂-CH₂-CH₂- and -CH₂-CH₂-CH₂-CH₂-CH₂-, etc. An "alkylene" may also be unsaturated.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002 ).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a particular embodiment of the invention, X-Y-Z in general formula (I) represent - N-N=CR₁ giving rise to general formula (Ia)

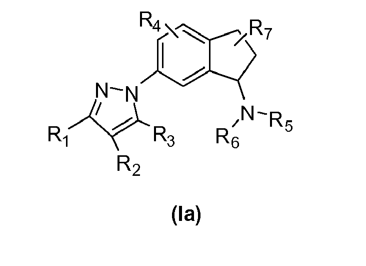

The meanings of R₁, R₂, R₃, R₄, R₅, R₆ and R₇ of formula (Ia) are identical to those defined above for formula (I), although, in a particular and preferred embodiment:
R₁ is selected from the group consisting of a linear or branched, saturated or unsaturated, optionally at least mono-substituted aliphatic radical;
R₂ and R₃ are independently from each other selected from the group consisting of hydrogen; or an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
R₄ is hydrogen;
R₅ and R₆ each are independently selected from the group consisting of a linear or branched, saturated or unsaturated, optionally at least mono-substituted aliphatic radical;
R₇ is a hydrogen
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Another particular embodiment of the invention arises when X-Y-Z in general formula I is represented by =C-NR²-N=. This substitution gives rise to compounds of general formula (Ib):

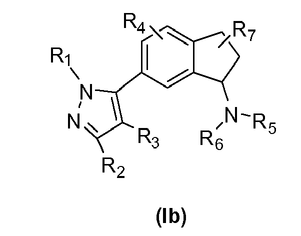

Again, the meanings of R₁, R₂, R₃, R₄, R₅, R₆ and R₇ in formula (Ib) are identical to those defined above for formula (I), although, in a particular and preferred embodiment:
R₁ is selected from the group consisting of a linear or branched, saturated or unsaturated, optionally at least mono-substituted aliphatic radical or a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group;
R₂ and R₃ are independently from each other selected from the group consisting of a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group; or an aliphatic radical, which is linear or branched, saturated or unsaturated, and optionally at least mono-substituted by F, Cl, Br, I, SH or OH;
or R₂ and R₃ form an saturated or unsaturated, optionally at least mono-substituted 5- or 6-membered-heterocyclic ring, which may be condensed with an optionally at least mono-substituted mono- or polycyclic ring system;
R₄ is hydrogen;
R₅ and R₆ each are independently selected from the group consisting of a linear or branched, saturated or unsaturated, optionally at least mono-substituted aliphatic radical or
R₇ is a hydrogen
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

The preferred compounds of the invention are selected from the following list:
[1] *N,N*-dimethyl-6-(3-methyl-1H-pyrazol-1-yl)-2,3-dihydro-1*H*-inden-1-amine
[2] 6-(3,5-dimethyl-1*H*-pyrazol-1-yl)-*N*,*N*-dimethyl-2,3-dihydro-1H-inden-1-amine
[3] *N*,*N*-dimethyl-6-(1,3,4-trimethyl-1*H*-pyrazol-5-yl)-2,3-dihydro-1*H*-inden-1-amine
[4] 6-(4-ethyl-1,3-dimethyl-1*H*-pyrazol-5-yl)-*N*,*N*-dimethyl-2,3-dihydro-1*H*-inden-1-amine
[5] *N*,*N*-dimethyl-6-(2-methyl-4,5,6,7-tetrahydro-2*H*-indazol-3-yl)-2,3-dihydro-1*H-*inden-1-amine
[6] 6-(1,3-dimethyl-4-phenyl-1*H*-pyrazol-5-yl)-*N*,*N*-dimethyl-2,3-dihydro-1*H*-inden-1-amine
[7] 6-(1-ethyl-3,4-dimethyl-1*H*-pyrazol-5-yl)-*N,N*-dimethyl-2,3-dihydro-1*H*-inden-1-amine
[8] 6-(3,4-dimethyl-1-phenyl-1*H*-pyrazol-5-yl)-*N*,*N*-dimethyl-2,3-dihydro-1*H*-inden-1-amine
[9] 6-(1-isopropyl-3,4-dimethyl-1*H*-pyrazol-5-yl)-*N*,*N*-dimethyl-2,3-dihydro-1*H*-inden-1-amine
[10] 6-(3,4-dimethyl-1-(2,2,2-trifluoroethyl)-1*H*-pyrazol-5-yl)-*N*,*N*-dimethyl-2,3-dihydro-1*H*-inden-1-amine

Another aspect of the present invention is the different processes for preparing the compounds of general formula I. Specifically, two different processes for preparing compounds of formula I are herein described referred as processes A and B.

### PROCESS A

Process A corresponds to a process for preparing compounds of formula I in the particular case that X-Y-Z is -N-N=CR₁, that is, a process for preparing compounds of general formula (Ia) comprising: 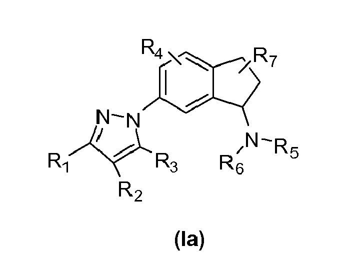
a) reacting a compound of general formula (II): 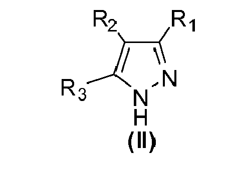 with a compound of general formula III:

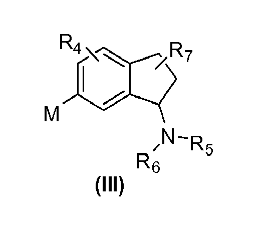

In the Ullmann reaction between compound (II) and (III) M represents a halogen atom, preferably a bromide, and R₁, R₂, R₃, R₄, R₅, R₆ and R₇ have the same meaning as in claim 1 or 2. The reaction is carried out in a suitable reaction medium and preferably in the presence of a copper catalyst, a suitable ligand and at least one base. This process can be performed by subjecting the reaction mixture to reflux by conventional heating for a period of time sufficient to achieve the title compound (Ia), or by microwave radiation.

Suitable reaction media are e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents preferably ethyl acetate, triethylamine, pyridine, dimethulsulfoxide, dimethylformamide, hexamethylphosphoramide, acetonitrile, acetone or nitromethane are included. Mixtures based one or more of the above mentioned solvents and water may also be used.

According to the invention, the bases that may be used in the process are generally organic or inorganic bases, preferably alkali metal hydroxides, e.g. sodium hydroxide or potassium hydroxide, or obtained from other metals such as barium hydroxide or different carbonates, preferably potassium carbonate, sodium carbonate, calcium carbonate or alkoxydes, e.g. sodium methoxide potassium methoxide, sodium ethoxide, potassium ethoxide or potassium tert-butoxide, or organic amines, preferably triethylamine, diisopropylethylamine or heterocycles, e.g. 1,4-diazabicyclo[2.2.2]octane, 1,8-diazabicyclo5.4.0]undec-7-ene, pyridine, diamino pyridine, dimethylaminopyridine, methylpiperidine or morpholine. Alkali metals such as sodium or its hydrides, e.g. sodium hydride, may also be used.

Ligand-free conditions are known for Ullmann coupling, but the reaction is preferably carried out in the presence of a suitable ligand such as mono- or bidentate ligands, such as phosphines, salicylamides, diamines, diols, amino alcohols, amino acids, phosphoramidites, oximephosphine, oxides, or phosphinidenes.

Preparation of compounds of general formula (III) can be achieved by nucleophilic substitution with amines of general formula (IV),

HNR₅R₆ **(IV)**

wherein R₅ and R₆ have the meaning given above, of the corresponding mesilate of compounds of general formula (V), 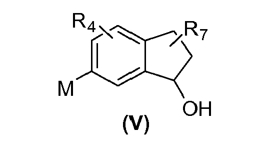 wherein R₄ and R₇ have the meaning given above and X represents halogen, preferably bromide.

Dihydroindenols of general formula (V) can be synthesized by treatment of compounds of general formula (VI) with a reducing agent in a suitable reaction media, 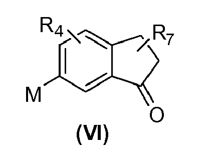 wherein R₄ and R₇ have the meaning given above and M represents halogen, preferably bromide.

Compounds of general formula (VI) can directly afford compounds of general formula (III) through a reductive amination with secondary amines of general formula (IV). The reductive amination is performed by reaction of a mixture comprising a compound of general formula (VI), and amino compound of general formula (IV) and a reducing agent in a suitable reaction medium, for a period of time sufficient to achieve the title compound (III). The reductive amination reaction can also be performed under microwave radiation. The use of microwave irradiation limits the formation of undesirable secondary reaction products, compared to what is obtained in a conventional reductive amination procedure. This process can be performed as a direct reaction when the carbonyl compound of general formula (VI) and the amine compound of general formula (IV) are mixed with the reducing agent without prior formation of the intermediate imine or iminium salt. A stepwise or indirect reaction involves the reduction of the preformatted imine in a separate step.

The choice of the reducing agent for reductive amination reaction can be conventionally made by those skilled in the art. Reducing agents useful in this procedure include hydrogen and a catalyst, zinc and HCl, sodium cyanoborohydride, lithium cyanoborohydride, tetrabutylammonium cyanoborohydride, cyanoborohydride on a solid support, sodium cyanoborohydride and dehydrating agents, sodium cyanoborohydride and titanium additives, sodium cyanoborohydride and zinc halide additives, sodium borohydride, sodium borohydride and dehydrating agents, sodium borohydride and titanium additives, sodium borohydride and zinc salt additives, lithium borohydride, potassium borohydride, polymer-supported borohydride, borohydride exchange resin with nickel acetate or palladium acetate, sodium triacetoxyborohydride, sodium triacetoxyborohydride and additives, tetramethylammonium triacetoxyborohydride, sodium cyano-9-borabicyclo[3.3.1]nonane, lithium triethylborohydride, lithium tri(sec-butyl)borohydride, sodium diisopinocampheylcyanoborohydride, amine boranes, borane-pyridine complex and alkylamine boranes. Sodium triacetoxyborohydride is particularly preferred because is non-toxic and generally does not reduce the carbonyl group prior to imine formation.

Suitable reaction media are the same as specified before.

The compounds of general formulas (II), (IV) and (VI) are either commercially available or can be produced according to methods known to those skilled in the art. The preparation of compounds of general formula (Ia) is illustrated in **scheme 1:**

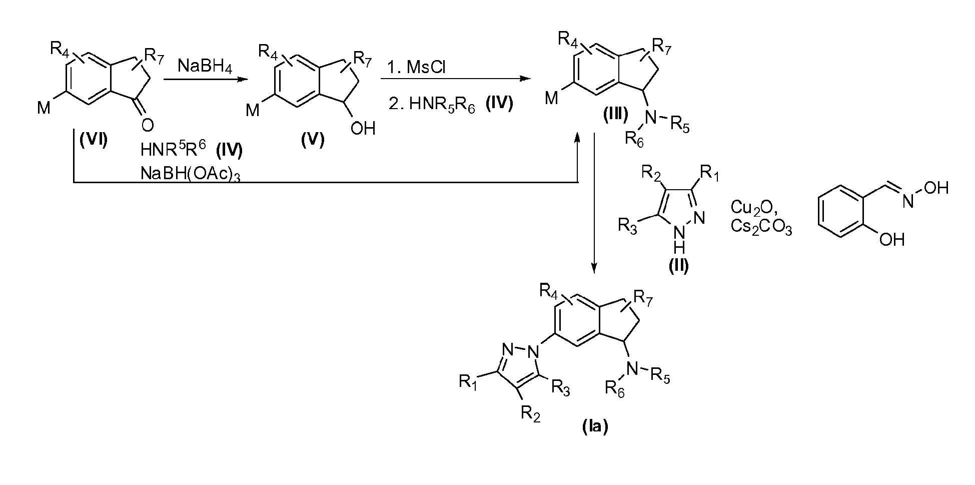

### PROCESS B

Process B corresponds to the preparation of compounds of formula I in the particular case that X-Y-Z is =C-NR²-N=. Compounds of formula (Ib) can be prepared by catalytic cross-coupling reactions which include the Kumada-Corriu-Tamao, Negishi, Stille, Hiyama, Suzuki-Miyaura, Heck, Sonogashira and other cross-coupling reactions known to those skilled in the art. More preferably, the compounds of general formula (Ib) can be prepared by cross-coupling Suzuki reaction.

Thus, process B is a process for the preparation of compounds of general formula (Ib) comprising: 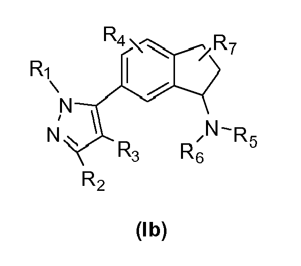
a) reacting a compound of general formula (VII): 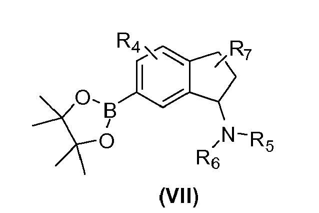 with a compound of general formula (VIII): 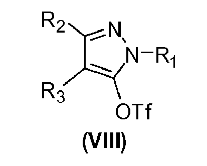 wherein M represents a halogen and R₁, R₂, R₃, R₄, R₅, R₆ and R₇ have the same meaning as in claims 1 or 3.

Process B is made in a suitable reaction medium, preferably in the presence of a palladium catalyst, a suitable ligand and at least one base. This process can be performed by subjecting the reaction mixture to reflux by conventional heating for a period of time sufficient to achieve the title compound (Ib), or by microwave radiation, preferably for 5 to 60 minutes, and at a temperature between 100 to 120°C.

Pyrazol compounds of general formula (VIII) can be synthesized from pyrazolones of general formula (IX), 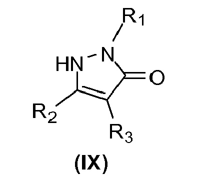 wherein R₁, R₂ and R₃ have the meaning given in claim 1 or 3, through reaction with trifluoromethanesulfonic anhydride in a suitable reaction medium, and in the presence of at least one base, preferably pyridine.

Compounds of general formula (IX) are obtained from 2-substituted acetoacetate compounds of general formula (X), 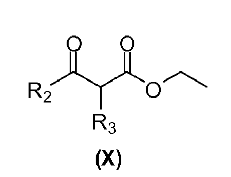 wherein R₂ and R₃ have the meaning given in claim 1 or 3, through ring closing reaction with hydrazines of general formula (XI),

R¹-NH-NH₂ **(XI)**

wherein R₁ have the meaning given in claim 1 or 3.

Preparation of boronate esters of general formula (VII) can be achieved from compounds of general formula (III), 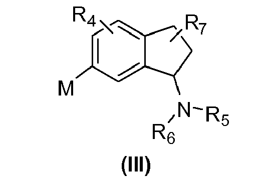 wherein R₄, R₅, R₆ and R₇ have the meaning given in claim 1 or 3, by treatment with a solution of BuLi followed by the addition of 2-isopropoxy-4,4,5,5-tetramethyl-1,3,2-dioxaborolane to the reaction mixture.

The synthesis of compounds of general formula (III) can be performed according to the methods described in process A and in scheme 1.

The compounds of general formulas (X) and (XI) are either commercially available or can be produced according to methods known to those skilled in the art.

Suitable reaction media are the same as expressed for process A.

The bases and reducing agents that may be used in the process are also the same as described before.

This method for the preparation of compounds of general formula (Ib) is illustrated in **scheme 2:**

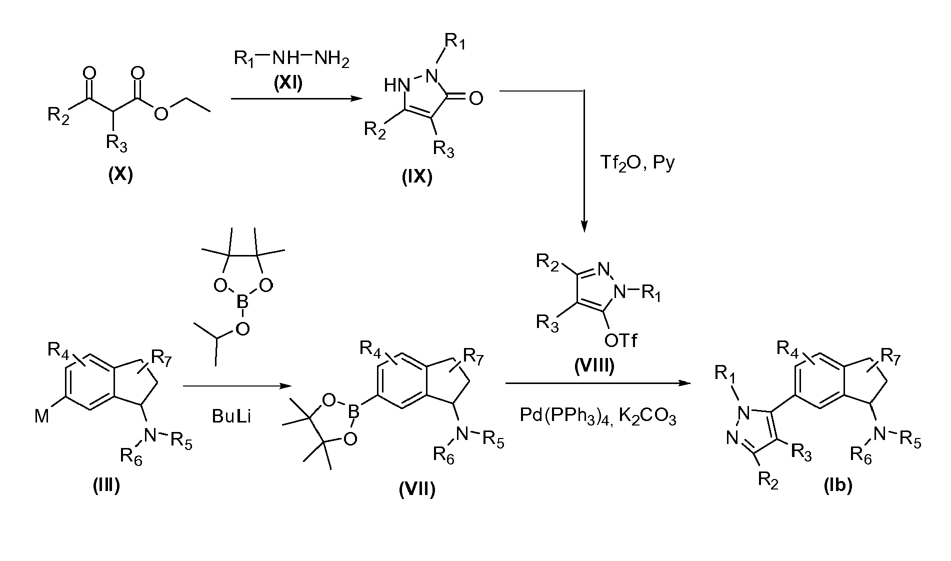

In a further aspect, the present invention also provides a process for the preparation of salts of compounds of general formula (I), wherein at least one compound of general formula (I) is reacted with an inorganic and/or organic acid, preferably in the presence of a suitable reaction medium. Suitable reaction media are the ones mentioned before. Suitable inorganic acid are for example hydrochloric acid, hydrobromic acid, phosphoric acid, sulphuric acid, nitric acid. Suitable organic acids are e.g. citric acid, maleic acid, fumaric acid, tartaric acid or derivatives thereof, such as p-toluenesulfonic acid, methanesulfonic acid or camphersulfonic acid.

In yet a further aspect the present invention also provides a process for the preparation of salts of compounds of general formula (I), wherein at least one compound of general formula (I) having at least one acidic group is reacted with one or more suitable bases, preferably in the presence of suitable reaction medium. Suitable bases are e.g. hydroxides. Carbonates or alkoxides, which include suitable cations, derived e.g. from alkaline metals, alkaline earth metals or organic cations, e.g. [NHnR₄₋ₙ]⁺ , wherein n is 0, 1, 2, 3 or 4 and R represents a branched or linear C1-4 alkyl radical.

Solvates, preferably hydrates, of the compounds of general formula (I), or corresponding stereoisomers, or corresponding salts may also be obtained by standard procedures known to those skilled in the art.

If the compounds of general formula (I) are obtained in form of a mixture of stereoisomers, particularly enantiomers or diastereomers, said mixtures may be separated by standard procedures known to those skilled in the art, e.g. chromatographic methods of crystallization with chiral reagents.

The purification and isolation of the compounds of general formula (I) or a corresponding stereoisomer, or a corresponding salt, or corresponding solvate respectively, if required may be carried out by conventional methods known to those skilled in the art, e.g. chromatographic methods or recrystallization.

The compounds of general formula (I), their stereoisomers or the respective salts or solvates are toxicologically acceptable and are therefore suitable as pharmaceutical active substances for the preparation of medicaments.

It is therefore another object of the invention to provide for a pharmaceutical formulation or medicament comprising at least one compound according to general formula I. In a particular embodiment, the formulation or medicament of the invention comprises at least a compound according to formula (Ia) or (Ib).

In a particular embodiment, the medicament/pharmaceutical composition according to the invention comprises at least one compound according to the invention, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

Furthermore, the present invention also provides for a pharmaceutical composition/medicament comprising at least one compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants, which is not yet formulated into a medicament.

The pharmaceutical composition/medicament of the invention is suitable for the treatment of a 5-HT7 mediated disease or condition, especially selected from pain, preferably visceral pain, chronic pain, cancer pain, migraine, acute pain or neuropathic pain, more prefearably neuropathic pain, allodynia or hyperalgesia or selected from sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

It is also an object of the invention the use of at least one compound according to any of claims 1 to 4 optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of 5-HT7 receptor mediated diseases or conditions such as those selected from pain, preferably visceral pain, chronic pain, cancer pain, migraine, acute pain or neuropathic pain, more prefearably neuropathic pain, allodynia or hyperalgesia or selected from sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

The medicament/pharmaceutical composition may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions of the present invention may also be administered topically or via a suppository.

The above mentioned compositions include preferably 1 to 60 % by weight of one or more of the compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, weight or degree of illness and so forth. The daily dosage for mammals including humans usally ranges from 1 milligram to 2000 milligram, preferably 1 to 1500 mg, more preferably 1 to 1000 mg of substance to be administered during one or several intakes.

Thus, the invention also provides a method of treatment using the medicament/pharmaceutical compositions described above.

The preparation of some compounds within the scope of the invention is illustrated in the following examples. These represent the preparation of some specific intermediates of the general processes A, B and C as well as compounds according to the general formula I obtained by such processes. These examples are given to illustrate the invention but they do not intend to be a limitation of it.

### EXAMPLES

### PROCESS A

### Example of compounds of general formula (V): 6-Bromo-2,3-dihydro-1H-inden-1-ol

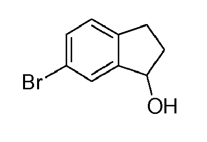

Under N₂ atmosphere, 6-bromo-1-indanone (1 mmol) was dissolved in MeOH (2.25 mL) and NaBH₄ (1 mmol) was added in 3 portions, every 10 min. After 30 min, the solvent was evaporated and crude was dissolved in EtOAc (1 mL) and H₂O (3 mL). The aqueous layer was extracted with EtOAc and the combined organic extracts were dried and concentrated to dryness to give pure alcohol in 96% yield.
¹H-NMR (400MHz, CDCl₃) δ ppm: 7.3-7.2 (m, 2H), 7.14 (dd, , *J*=8 and 2 Hz, 1H), 5.26 (t, *J*=6 Hz, 1H,), 3.09-3.01 (m, 1H), 2.87-2.80 (m, 1H), 2.62-2.54 (m, 1H), 2.03-1.96 (m, 1H).

### Example of compounds of general formula (III): 6-Bromo-2,3-dihydro-N,N-dimethyl-1H-inden-1-amine

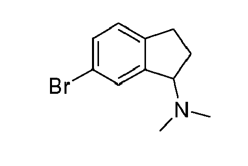

This compound can be synthesized in three different ways :
**(a)** A solution of 6-bromo-indan-1-ol (1 mmol) and NEt₃ (4 mmol) in dry THF (5 mL) was stirred at -15 °C under argon. A solution of methanesulfonyl chloride (2 mmol) in dry THF (1 mL) was cooled to -78 °C and then slowly added to the alcohol solution maintaining the temperature below 0 °C. The reaction mixture was stirred for 2h at -15 °C and then purged with dimethylamine gas (12 mmol). The reaction mixture was allowed to warm to room temperature and stirred overnight. Then, it was filtered to remove salts and solvent was evaporated. Crude was dissolved in CH₂Cl₂ and extracted with HCl 1 M (3x). The aqueous phase was basified with NaOH 6M to pH 8-9 and extracted with CH₂Cl₂. The combined organic extracts were dried over MgSO₄ and concentrated to dryness. The crude was used in next step without further purification.
**(b)** 6-Bromo-indan-1-one (0.23 mmol) and dimethyl-amine (0.71 mmol) were mixed in 3 ml of 1,2-dichloroethane in a process vial, which was sealed with a septum. Sodium triacetoxyborohydride (0.47 mmol) was added under argon atmosphere. The suspension was subjected to microwave irradiating conditions (CEM Discover^{®} equipped with a CEM Explorer^{®} automated reaction handling module). The reaction mixture was heated for 5 min at 120°C and then cooled. The crude was evaporated to dryness and then suspended in aqueous NaHCO₃. The product was extracted with CH₂Cl₂ and washed with aqueous NaHCO₃. The CH₂Cl₂ extract was dried with anhydrous Na₂SO₄, filtered and evaporated to dryness to give the crude product 6-bromo-2,3-dihydro-*N*,*N*-dimethyl-1*H*-inden-1-amine. The crude was purified by flash column chromatography (CH₂Cl₂-MeOH as eluents) by using a CombiFlash Companion™ system to yield the title compound (85%).
**(c)** 6-Bromo-indan-1-one (0.58 mmol) was dissolved in MeOH. Dimethyl-amine (3.7 mmol), acetic acid (0.059 mmol) and sodium cyanoborohydride (1.74 mmol) were added under argon atmosphere. The suspension was refluxed overnight and then cooled. The crude was evaporated to dryness and then suspended in water. The product was extracted with CH₂Cl₂ and washed with water. The CH₂Cl₂ extract was dried with anhydrous Na₂SO₄, filtered and evaporated to dryness to give the crude product (6-bromo-indan-1-yl)-dimethyl-amine (80% conversion LC-MS). The crude product was used in the next synthetic step.
¹H-NMR (400MHz, CDCl₃) δ ppm: 7.49 (s, 1H), 7.32 (dd, *J*=8 and 2 Hz, 1H), 7.07 (d, *J*=8 Hz, 1H), 4.30 (t, *J*=7 Hz, 1H), 2.91-2.83 (m, 1H), 2.79-2.71 (m, 1H), 2.25 (s, 6H), 2.09-2.03 (m 2H).

### Example 1: N,N-dimethyl-6-(3-methyl-1H-pyrazol-1-yl)-2,3-dihydro-1H-inden-1-amine dihydrochloride

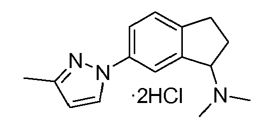

To a sealed tube containing 3-methylpyrazole (1 mmol), Cu₂O (0.04 mmol), Cs₂CO₃ (1.6 mmol) and salicylaldoxime (0.16 mmol), a solution of 6-bromo-2,3-dihydro-*N,N-*dimethyl-1*H*-inden-1-amine (1 mmol) in acetonitrile (0.6 mL) was added and the resulting suspension was heated at 120 °C for 4h under argon atmosphere. Then, a second portion of CuO₂ (0.04 mmol) and Cs₂CO₃ (0.04 mmol) were added and heated at 120 °C for 5 days. Then, the suspension was filtered trough a pad of Celite, washed with CH₂Cl₂ and concentrated. Crude was redissolved in CH₂Cl₂ and washed with water (3x), the organic phase was dried and evaporated to give an oil which contains starting material and final product (as a mixture of the two possible regioisomers in a 4:1 ratio) in a 3:10 ratio. The crude was purified by flash chromatography (with neutral Al₂O₃; using cyclohexane and ethyl acetate as eluents). A second flash chromatography was needed (SiO₂ with CH₂Cl₂-methanol) to obtain pure product in 17% overall yield. The amine was transformed into the corresponding dihydrochloride.

### Example 2: 6-(3,5-Dimethyl-1H-pyrazol-1-yl)-N,N-dimethyl-2,3-dihydro-1H-inden-1-amine dihydrochloride

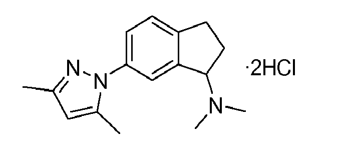

To a sealed tube containing 3,5-dimethylpyrazole (1 mmol), Cul (0.2 mmol), K₂CO₃ (2 mmol) and trans-1,2-diaminocyclohexane (0.2 mmol) and a solution of 6-bromo-2,3-dihydro-*N,N*-dimethyl-1*H*-inden-1-amine (1 mmol) in distilled DMF (0.6 mL) was added and the resulting suspension was heated at 150 °C under Ar atmosphere for 5 days. The work-up was performed as described above for Example 1. The crude was purified by flash chromatography (with neutral Al₂O₃, using cyclohexane and ethyl acetate as eluents). A second chromatography was needed (SiO₂ with CH₂Cl₂-methanol) to obtain pure product in 21% overall yield. The amine was transformed into the corresponding dihydrochloride.

### PROCESS B

### Example of compounds of general formula (VII): 2,3-Dihydro-N,N-dimethyl-6-(4,4,5,5-tetramethyl-1,3,2-dioxaborolan-2-yl)-1H-inden-1-amine

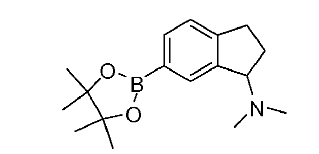

Under N₂ atmosphere, 6-bromo-2,3-dihydro-*N,N*-dimethyl-1*H*-inden-1-amine (4.16 mmol) was dissolved in anhydrous THF (24 mL) and cooled to -78 °C. 1.6M BuLi solution in hexanes (5.4 mmol) was added dropwise and the reaction mixture was stirred at this temperature for 45min. Then, 2-isopropoxy-4,4,5,5-tetramethyl-1,3,2-dioxaborolane (5.4 mmol) was added. After 2h, the reaction mixture was allowed to reach room temperature and poured onto water (30 mL). Solvent was concentrated and the aqueous phase was extracted with CH₂Cl₂ (3 x 15 mL). The combined organic layers were washed with brine (1 x 20 mL), dried (MgSO₄) and concentrated in vacuo to afford 0.962 mg (80% yield) of an oil which was used in next step without further purification.

### Example of compounds of general formula (IX): 1,2-Dihydro-2,4,5-trimethylpyrazol-3-one hydrochloride

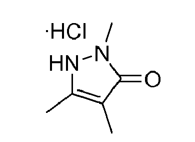

Under N₂ atmosphere, ethyl-2-methyl-acetoacetate (1 mmol) was dissolved in toluene (1.5 mL) and EtOH (0.1 mL). Acetic acid (30 µL) was added and the solution was cooled to 0 °C. Methylhydrazine (1.5 mmol) was added dropwise and the resulting solution was stirred at room temperature 30 min and then heated at 45 °C o/n. EtOH was evaporated and the organic solution was extracted with 1 M HCl (3x). Then, the aqueous layer was concentrated until a solid was formed. The suspension was stirred at 0 °C for 1h and the solid was filtered and dried in vacuo to yield 73% of pyrazol-3-one hydrochloride.
¹H-NMR (400MHz, MeOD) δ ppm: 3.70 (s, 3 H), 2.27 (s, 3 H), 1.95 (s, 3 H).

### Example of compounds of general formula (VIII): 1,3,4-Trimethyl-1H-pyrazol-5-yl trifluoromethanesulfonate

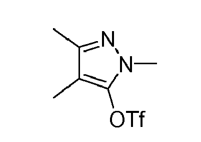

To a cooled solution (at 0 °C) of 1,2-dihydro-2,4,5-trimethylpyrazol-3-one hydrochloride (1 mmol) and pyridine (1.5 mmol) in distilled CH₂Cl₂ was added Tf₂O (1.1 mmol) dropwise. After 30 min at 0 °C and 30 min at room temperature, the reaction mixture was poured into H₂O-ice (6 mL) and extracted with CH₂Cl₂ (2x). The combined organic extracts were washed with brine, dried and concentrated in vacuo at 10-15 °C. Crude was purified by flash chromatography (SiO₂, CyclohexaneEtOAc) to yield 67% of pure compound.
¹H-NMR (400MHz, CDCl₃) δ ppm: 3.72 (s, 3 H), 2.17 (s, 3 H), 1.95 (s, 3 H).

### Example 3: N,N-dimethyl-6-(1,3,4-trimethyl-1H-pyrazol-5-yl)-2,3-dihydro-1H-inden-1-amine

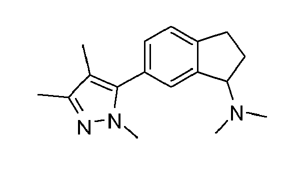

1,3,4-trimethyl-1*H*-pyrazol-5-yl trifluoromethanesulfonate (1.63 mmol) and K₂CO₃ (3.7 mmol) were dissolved in 1,2-dimethoxyethane (DME) (19 mL) and water (2.4 mL) and the solution was degassed with argon. Then, 2,3-dihydro-*N,N*-dimethyl-6-(4,4,5,5-tetramethyl-1,3,2-dioxaborolan-2-yl)-1*H*-inden-1-amine (1.63 mmol) and Pd(PPh₃)₄ (0.04 mmol) were added. The reaction mixture was heated at 85 °C until starting material was not detected by TLC (2.5h). It was allowed to cool down to room temperature and evaporated to dryness. The crude was dissolved with CH₂Cl₂ and filtered trough a pad of Celite. The organic phase was extracted with HCl 6M (3x), the combined aqueous layer was washed with CH₂Cl₂ and basified to pH= 8-9. The basic aqueous phase was extracted with CH₂Cl₂ (3x) dried and concentrated. Purification by flash chromatography (SiO₂/NEt₃ 2.5% v/v, CH₂Cl₂/MeOH) afforded pure compound in 25% yield. The compound was converted into its corresponding hydrochloride.

### Example 4: 6-(4-Ethyl-1,3-dimethyl-1H-pyrazol-5-yl)-N,N-dimethyl-2,3-dihydro-1H-inden-1-amine

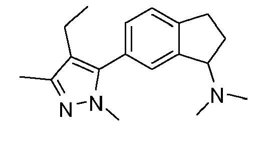

Example compound 4 was prepared following the experimental procedure described for example compound 3 using: 4-ethyl-1,3-dimethyl-1*H*-pyrazol-5-yl trifluoromethanesulfonate (1.8 mmol), K₂CO₃ (3.5 mmol), 2,3-dihydro-*N,N*-dimethyl-6-(4,4,5,5-tetramethyl-1,3,2-dioxaborolan-2-yl)-1*H*-inden-1-amine (1.8 mmol), Pd(PPh₃)₄ (0.045 mmol) in 1,2-dimethoxyethane (DME) (18 mL) and water (2.3 mL). After flash chromatography (Al₂O₃, Cyclohexane/EtOAc), 95 mg of title compound were obtained (20% yield). The compound was converted into its corresponding hydrochloride.

### Example 5: N,N-dimethyl-6-(2-methyl-4,5,6,7-tetrahydro-2H-indazol-3-yl)-2,3-dihydro-1H-inden-1-amine

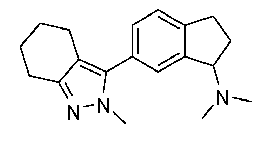

Under argon atmosphere, 4,5,6,7-tetrahydro-2-methyl-2*H*-indazol-3-yl trifluoromethane sulfonate (0.35mmol) was dissolved in toluene:ethanol (1:1, 5 mL). A solution of 2,3-dihydro-*N,N*-dimethyl-6-(4,4,5,5-tetramethyl-1,3,2-dioxaborolan-2-yl)-1*H*-inden-1-amine (0.35 mmol) in toluene (2 mL) was added, followed by 2M solution of Na₂CO₃ (1.19 mmol). Pd(PPh₃)₄ (0.06 mmol) was added and the reaction mixture was heated at reflux temperature for 20 h. The solvent was evaporated, crude was redissolved in EtOAc and washed with H₂O (3x) and brine (1x). The organic layer was extracted with 6M HCl (1x) and 3M HCl (2x). The aqueous phase was basified to pH 9 and extracted with CH₂Cl₂ (3x). The combined organic extracts were dried (MgSO₄) and concentrated to dryness. Purification by flash chromatography (SiO₂, CH₂Cl₂/MeOH) afforded title compound in 46% yield. The compound was converted into its corresponding hydrochloride.

### Example 6: 6-(1,3-Dimethyl-4-phenyl-1H-pyrazol-5-yl)-N,N-dimethyl-2,3-dihydro-1H-inden-1-amine

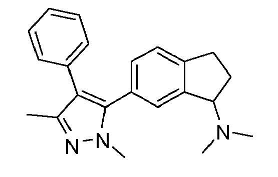

Example compound 6 was prepared following the experimental procedure described for example compound 5, using: 4-phenyl-1,3-dimethyl-1*H*-pyrazol-5-yl trifluoromethanesulfonate (1.25 mmol) in toluene:ethanol (1:1, 10 mL), 2,3-dihydro-*N,N*-dimethyl-6-(4,4,5,5-tetramethyl-1,3,2-dioxaborolan-2-yl)-1*H*-inden-1-amine
(1.25 mmol) in toluene (5 mL), Pd(PPh₃)₄ (0.22 mmol) and Na₂CO₃ (4.24 mmol). After flash chromatography (SiO₂, CH₂Cl₂/MeOH), 136 mg of the title compound were obtained (33% yield).

### Example 7: 6-(1-Ethyl-3,4-dimethyl-1H-pyrazol-5-yl)-N,N-dimethyl-2,3-dihydro-1H-inden-1-amine

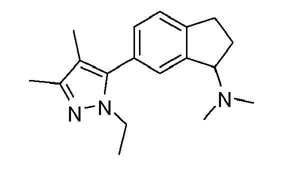

Example compound 7 was prepared following the experimental procedure described for example compound 5, using: 1-ethyl-3,4-dimethyl-1*H*-pyrazol-5-yl trifluoromethanesulfonate (1.22 mmol) in toluene:ethanol (1:1, 10 mL), 2,3-dihydro-*N,N*-dimethyl-6-(4,4,5,5-tetramethyl-1,3,2-dioxaborolan-2-yl)-1*H*-inden-1-amine (1.22 mmol) in toluene (5 mL), Pd(PPh₃)₄ (0.21 mmol) and Na₂CO₃ (4.14 mmol). After flash chromatography (SiO₂/NEt₃ 2.5% v/v, CH₂Cl₂/MeOH), 94 mg of pure title compound were obtained (26% yield). The compound was converted into its corresponding hydrochloride.

### Example 8: 6-(3,4-Dimethyl-1-phenyl-1H-pyrazol-5-yl)-N,N-dimethyl-2,3-dihydro-1H-inden-1-amine

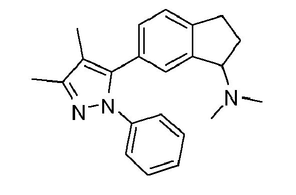

Example compound 8 was prepared following the experimental procedure described for example compound 5, using: 1-phenyl-3,4-dimethyl-1*H*-pyrazol-5-yl trifluoromethanesulfonate (0.83 mmol) in toluene:ethanol (1:1, 8 mL), 2,3-dihydro-*N,N*-dimethyl-6-(4,4,5,5-tetramethyl-1,3,2-dioxaborolan-2-yl)-1*H*-inden-1-amine (0.83 mmol) in toluene (4 mL), Pd(PPh₃)₄ (0.21 mmol) and Na₂CO₃ (2.84 mmol). After flash chromatography (SiO₂/NEt₃ 2.5% v/v, EtOAc and an additional purification with SiO₂, CH₂Cl₂/MeOH), 47 mg of pure title compound were obtained (17% yield). The compound was converted into its corresponding hydrochloride.

### Example 9: 6-(1-Isopropyl-3,4-dimethyl-1H-pyrazol-5-yl)-N,N-dimethyl-2,3-dihydro-1H-inden-1-amine

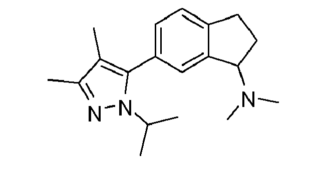

Example compound 9 was prepared following the experimental procedure described for example compound 5, using: 1-isopropyl-3,4-dimethyl-1*H*-pyrazol-5-yl trifluoromethanesulfonate (0.99 mmol) in toluene:ethanol (1:1, 5 mL), 2,3-dihydro-*N,N*-dimethyl-6-(4,4,5,5-tetramethyl-1,3,2-dioxaborolan-2-yl)-1*H*-inden-1-amine (0.99 mmol) in toluene (5 mL), Pd(PPh₃)₄ (0.17 mmol) and Na₂CO₃ (3.37 mmol). After flash chromatography (SiO₂, CH₂Cl₂/MeOH), 88 mg of the title compound were obtained (30% yield). The compound was converted into its corresponding hydrochloride.

### Example 10: 6-(3,4-Dimethyl-1-(2,2,2-trifluoroethyl)-1H-pyrazol-5-yl)-N,N-dimethyl-2,3-dihydro-1H-inden-1-amine

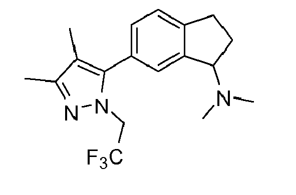

Example compound 10 was prepared following the experimental procedure described for example compound 5, using: 1-(2,2,2-trifluoroethyl)-3,4-dimethyl-1*H-*pyrazol-5-yl trifluoromethanesulfonate (1.04 mmol) in toluene:ethanol (1:1, 5 mL), 2,3-dihydro-*N,N*-dimethyl-6-(4,4,5,5-tetramethyl-1,3,2-dioxaborolan-2-yl)-1*H*-inden-1-amine (1.04 mmol) in toluene (5 mL), Pd(PPh₃)₄ (0.18 mmol) and Na₂CO₃ (3.55 mmol). After flash chromatography (SiO₂, CH₂Cl₂/MeOH), 101 mg of the title compound were obtained (29% yield). The compound was converted into its corresponding hydrochloride.

The following table shows the espectroscopic data of the examples 1-10 :

| **Example** | **Structure** | **Name** | **¹H-NMR** |
|---|---|---|---|
| 1 | 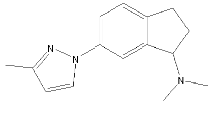 | *N,N*-dimethyl-6-(3-methyl-1H-pyrazol-1-yl)-2,3-dihydro-1*H*-inden-1-amine | ¹H-NMR (400MHz, METHANOL-*d*₄) δ ppm: 8.12 (b, 1H), 7.90 (b, 1H), 7.76 (dd, *J*=8 and 2 Hz, 1H), 7.50 (d, *J*=8 Hz, 1H), 6.53 (b, 1H), 5.07 (m, 1H), 3.25-3.15 (m, 1H), 3.10-3.03 (m, 1H), 2.9 (s,3H), 2.74 (s, 3H), 2.64-2.54 (m 1H), 2.52-2.45 (m, 1H), 2.35 (s, 3H). (hydrochloride product) |
| 2 | 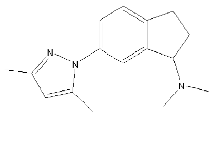 | 6-(3,5-dimethyl-1H-pyrazol-1-yl)-*N,N*-dimethyl-2,3-dihydro-1H-inden-1-amine | ¹H-NMR (400 MHz, METHANOL-*d*₄) δ ppm: 7.89 (s, 1H), 7.66-7.60 (m, 2H), 6.44 (s, 1H), 5.14 (dd, *J*=9 and 4 Hz, 1H), 3.33-3.24 (m, 1H), 3.18-3.10 (m, 1H), 2.92 (s, 3H), 2.73 (s, 3H), 2.68-2.60 (m 1H), 2.58-2.50 (m, 1H), 2.42 (s, 3H), 2.36 (s, 3H). (hydrochloride product) |
| 3 | 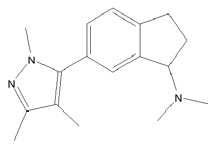 | *N,N*-dimethyl-6-(1,3,4-trimethyl-1*H*-pyrazol-5-yl)-2,3-dihydro-1*H*-inden-1-amine | ¹H NMR (400 MHz, METHANOL-*d*₄) δ ppm: 1.94 (s, 3H), 2.22 (s, 3H), 2.47-2.68 (m, 2H), 2.73 (s, 3H), 2.92 (s, 3H), 3.08-3.29 (m, 2H), 3.69 (s, 3H), 5.09 (dd, *J*=8.21, 3.13 Hz, 1H), 7.45 (dd, *J*=7.82, 1.56 Hz, 1H), 7.58 (d, *J*=7.82 Hz, 1H), 7.59 (s, 1H). (hydrochloride product) |
| 4 | 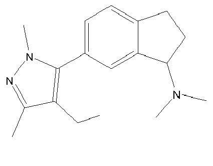 | 6-(4-ethyl-1,3-dimethyl-1*H*-pyrazol-5-yl)-*N,N*-dimethyl-2,3-dihydro-1*H-*inden-1-amine | ¹H NMR (400 MHz, METHANOL-*d*₄) δ ppm: 1.00 (t, *J*=7.62 Hz, 3H), 2.23 (s, 3H), 2.37 (q, *J*=7.69 Hz, 2H), 2.46-2.66 (m, 2H), 2.70-2.93 (m, 6H), 3.08-3.29 (m, 2H), 3.63 (s, 3H), 5.08 (dd, *J*=8.21, 3.13 Hz, 1H), 7.44 (dd, *J*=7.82, 1.56 Hz, 1H), 7.55 (s, 1H), 7.57 (d, *J*=7.82 Hz, 1H). (hydrochloride product) |
| 5 | 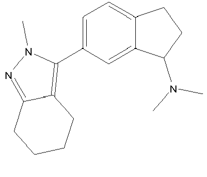 | *N,N*-dimethyl-6-(2-methyl-4,5,6,7-tetrahydro-2*H*-indazol-3-yl)-2,3-dihydro-1*H*-inden-1-amine | ¹H NMR (400 MHz, METHANOL-*d*₄) δ ppm: 1.72-1.80 (m, 2H), 1.81-1.89 (m, 2H), 2.48 (t, *J*=6.06 Hz, 2H), 2.50-2.63 (m, 2H), 2.66 (t, *J*=6.25 Hz, 2H), 2.73 (s, 3H), 2.92 (s, 3H), 3.07-3.28 (m, 2H), 3.75 (s, 3H), 5.08 (dd, *J*=8.21, 3.13 Hz, 1H), 7.49 (dd, *J*=7.82, 1.56 Hz, 1H), 7.56 (d, *J*=7.82 Hz, 1H), 7.61 (s, 1H). (hydrochloride product) |
| 6 | 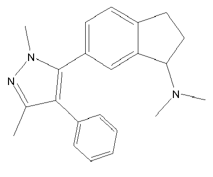 | 6-(1,3-dimethyl-4-phenyl-1*H*-pyrazol-5-yl)-*N,N*-dimethyl-2,3-dihydro-1*H*-inden-1-amine | ¹H NMR (400 MHz, METHANOL-*d*₄) δ ppm: 2.27 (s, 3H), 2.30 - 2.55 (m, 2H), 2.50 (s, 6H), 2.95-3.24 (m, 2H), 3.77 (s, 3H), 4.80 (dd, *J*=8.21, 3.52 Hz, 1H) 7.08-7.11 (m, 2H), 7.17-7.28 (m, 3H), 7.30 (s, 1H), 7.43 (dd, *J*=7.82, 1.56 Hz, 1H), 7.48 (d, *J*=7.82 Hz, 1H). |
| 7 | 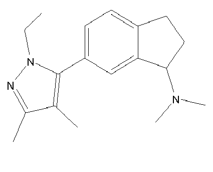 | 6-(1-ethyl-3,4-dimethyl-1*H*-pyrazol-5-yl)-*N,N*-dimethyl-2,3-dihydro-1*H-*inden-1-amine | ¹H NMR (400 MHz, METHANOL-*d*₄)δ ppm: 1.33 (t, *J*=7.42 Hz, 3H), 1.97 (s, 3H), 2.35 (s, 3H), 2.48-2.66 (m, 2H), 2.71 (s, 3H), 2.93 (s, 3H), 3.10-3.29 (m, 2H), 4.14 (q, *J*=7.42 Hz, 2H), 5.13 (dd, *J*=8.21, 3.52 Hz, 1H), 7.50 (dd, *J*=7.82, 1.56 Hz, 1H), 7.63 (d, *J*=7.82 Hz, 1H), 7.73 (s, 1H). (hydrochloride product) |
| 8 | 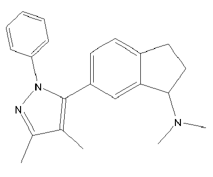 | 6-(3,4-dimethyl-1-phenyl-1*H*-pyrazol-5-yl)-*N,N*-dimethyl-2,3-dihydro-1*H*-inden-1-amine | ¹H NMR (400 MHz, METHANOL-*d*₄) δ ppm: 2.05 (s, 3H), 2.31 (s, 3H), 2.32-2.50 (m, 2H), 2.52 (s, 6H), 2.95-3.24 (m, 2H), 4.82 (dd, *J*=8.60, 3.52 Hz, 1H), 7.18-7.21 (m, 2H), 7.24 (s, 1H), 7.26-7.36 (m, 3H), 7.37 (dd, *J*=8.21, 1.56 Hz, 1H), 7.45 (d, *J*=7.82 Hz, 1H). (hydrochloride product) |
| 9 | 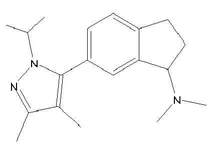 | 6-(1-isopropyl-3,4-dimethyl-1*H*-pyrazol-5-yl)-*N,N*-dimethyl-2,3-dihydro-1*H-*inden-1-amine | ¹H NMR (400 MHz, METHANOL-*d*₄)δ ppm: 1.51 (d, *J*=2.34 Hz, 3H), 1.53 (d, *J*=2.34 Hz, 3H), 1.99 (s, 3H), 2.45 (s, 3H), 2.49-2.69 (m, 2H), 2.71 (s, 3H), 2.94 (s, 3H), 3.11-3.29 (m, 2H), 4.54-4.70 (m, *J*=6.64, 6.64, 6.64, 6.64, 6.64, 6.64 Hz, 1H), 5.14 (dd, *J*=8.21, 3.52 Hz, 1H), 7.52 (dd, *J*=7.82, 1.56 Hz, 1H), 7.67 (d, *J*=7.82 Hz, 1H), 7.80 (s, 1H). (hydrochloride product) |
| 10 | 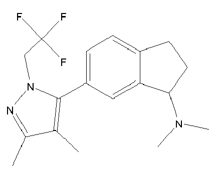 | 6-(3,4-dimethyl-1-(2,2,2-trifluoroethyl)-1*H*-pyrazol-5-yl)-*N,N*-dimethyl-2,3-dihydro-1*H*-inden-1-amine | ¹H NMR (400 MHz, METHANOL-*d*₄) δ ppm: 1.91 (s, 3H), 2.16-2.23 (m, 2H), 2.24 (s, 3H), 2.27 (s, 6H), 2.89-3.10 (m, 2H), 4.42 (t, *J*=6.64 Hz, 1H), 4.65 (dq, *J*=8.21, 3.52 Hz, 2H), 7.19 (dd, *J*=7.82, 1.56 Hz, 1H), 7.34 (s, 1H), 7.41 (d, *J*=7.82 Hz, 1H). |

### PHARMACOLOGICAL DATA

### Radioligand binding

Radioligand binding assays were performed using the Cloned Human Serotonin Receptor, Subtype 7 (h5HT7), expressed in CHO cells, coated on Flashplate (Basic FlashPlate Cat.: SMP200) from PerkinElmer (Cat.: 6120512). The protocol assay was essentially the recommended protocol in the Technical Data Sheet by PerkinEmer Life and Analytical Sciences. The Mass membrane protein/well was typically 12 g and the Receptor/well was about 9-10 fmoles. The Flashplate were let equilibrate at room temperature for one hour before the addition of the components of the assay mixture. The binding buffer was: 50 mM Tris-HCl, pH 7.4, containing 10 mM MgCl2, 0.5 mM EDTA and 0.5% BSA. The radioligand was [¹²⁵I]LSD at a final concentration of 0.82 nM. Nonspecific binding was determined with 50 M of Clozapine. The assay volume was 25 I. TopSeal-A were applied onto Flashplate microplates and they were incubated at room temperature for 240 minutes in darkness. The radioactivity were quantified by liquid scintillation spectrophotometry (Wallac 1450 Microbeta Trilux) with a count delay of 4 minutes prior to counting and a counting time of 30 seconds per well. Competition binding data were analyzed by using the LIGAND program (Munson and Rodbard, LIGAND: A versatile, computerized approach for characterization of ligandbinding systems. Anal. Biochem. 107: 220-239, 1980 ) and assays were performed in triplicate determinations for each point.

Results of the radioligand assay for representative compounds/examples are given in the table below:

| **Example** | **Structure** | **5-HT₇ Ki (nM)** |
|---|---|---|
| 3 | 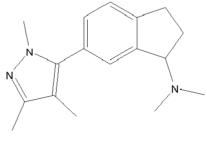 | 425.2 |
| 9 | 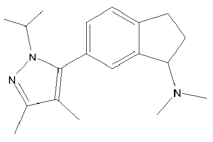 | 360.3 |

### FORMULATION EXAMPLE

### Example of a tablet formulation:

Compound according to example 3: 5 mg,
Lactose:_ 60 mg
Crystalline cellulose: 25 mg
Povidone K 90: 5 mg
Pregelanitized starch: 3 mg
Colloidal silica dioxide: 1 mg
Magnesium stearate: 1 mg
Total weight per tablet: 100 mg

The above mentioned ingredients were mixed and compressed into a tablet by conventional methods known to those skilled in the art.

## Claims

1. A compound of general formula (I) 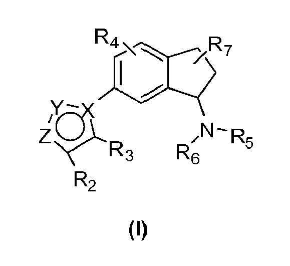 wherein
X-Y-Z together form
-N-N=CR₁- or =C-NR₁-N= ;
wherein
R₁ is selected from the group consisting of hydrogen, a linear or branched, saturated or unsaturated, optionally at least mono-substituted aliphatic radical or a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group;
R₂ and R₃ are independently from each other selected from the group consisting of hydrogen; a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group; or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH or R₂ and R₃ form an saturated or unsaturated, optionally at least mono-substituted 5- or 6-membered-heterocyclic ring, which may be condensed with an optionally at least mono-substituted mono- or polycyclic ring system;
R₄ is selected from hydrogen, halogen, OH, SH, NH_{2,} a linear or branched, saturated or unsaturated, optionally at least mono-substituted aliphatic radical or O-R with R being a linear or branched, saturated or unsaturated, optionally at least mono-substituted aliphatic radical;
R₅ and R₆ each are independently selected from the group consisting of hydrogen, a linear or branched, saturated or unsaturated, optionally at least mono-substituted aliphatic radical or
R₅ and R₆ together with the bridging nitrogen atom form a saturated or unsaturated, optionally at least mono-substituted 5- or 6-membered-heterocyclic ring, which may be condensed with an optionally at least mono-substituted mono- or polycyclic ring system;
R₇ is a hydrogen
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

2. A compound according to claim 1 having a general formula (Ia): 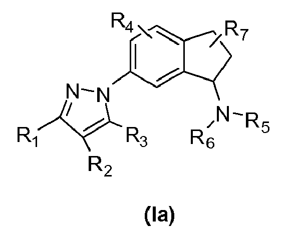 wherein
R₁ is selected from the group consisting of a linear or branched, saturated or unsaturated, optionally at least mono-substituted aliphatic radical;
R₂ and R₃ are independently from each other selected from the group consisting of hydrogen; or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R₄ is hydrogen;
R₅ and R₆ each are independently selected from the group consisting of a linear or branched, saturated or unsaturated, optionally at least mono-substituted aliphatic radical;
R₇ is hydrogen
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

3. A compound according to claim 1 having a general formula (Ib) 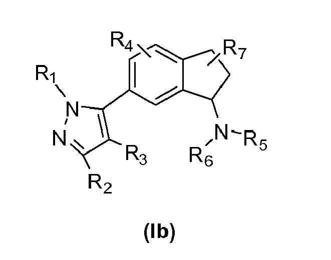 wherein
X-Y-Z together form
-N-N=CR¹- or =C-NR¹-N= ;
wherein
R₁ is selected from the group consisting of a linear or branched, saturated or unsaturated, optionally at least mono-substituted aliphatic radical or a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group;
R₂ and R₃ are independently from each other selected from the group consisting of a phenyl radical, optionally at least mono-substituted by F, Cl, Br, I, SH, OH or O-R with R being an aliphatic radical, which is linear or branched and optionally at least mono-substituted by F, Cl, Br, I, SH or OH and which may be bonded by an alkylene group; or an aliphatic radical, which is linear or branched, saturated or unsaturated, and optionally at least mono-substituted by F, Cl, Br, I, SH or OH;
or R₂ and R₃ form an saturated or unsaturated, optionally at least mono-substituted 5- or 6-membered-heterocyclic ring, which may be condensed with an optionally at least mono-substituted mono- or polycyclic ring system;
R₄ is hydrogen;
R₅ and R₆ each are independently selected from the group consisting of a linear or branched, saturated or unsaturated, optionally at least mono-substituted aliphatic radical or
R₇ is a hydrogen
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

4. A compound according to claim 1 selected from:
[1] *N*,*N*-dimethyl-6-(3-methyl-1H-pyrazol-1-yl)-2,3-dihydro-1*H*-inden-1-amine
[2] 6-(3,5-dimethyl-1*H*-pyrazol-1-yl)-*N,N*-dimethyl-2,3-dihydro-1*H*-inden-1-amine
[3] *N,N*-dimethyl-6-(1,3,4-trimethyl-1*H*-pyrazol-5-yl)-2,3-dihydro-1*H*-inden-1-amine
[4] 6-(4-ethyl-1,3-dimethyl-1*H*-pyrazol-5-yl)-*N,N*-dimethyl-2,3-dihydro-1*H*-inden-1-amine
[5] *N,N*-dimethyl-6-(2-methyl-4,5,6,7-tetrahydro-2H-indazol-3-yl)-2,3-dihydro-1*H-*inden-1-amine
[6] 6-(1,3-dimethyl-4-phenyl-1*H*-pyrazol-5-yl)-*N,N*-dimethyl-2,3-dihydro-1*H*-inden-1-amine
[7] 6-(1-ethyl-3,4-dimethyl-1*H*-pyrazol-5-yl)-*N,N*-dimethyl-2,3-dihydro-1*H*-inden-1-amine
[8] 6-(3,4-dimethyl-1-phenyl-1*H*-pyrazol-5-yl)-*N,N*-dimethyl-2,3-dihydro-1*H*-inden-1-amine
[9] 6-(1-isopropyl-3,4-dimethyl-1*H*-pyrazol-5-yl)-*N,N*-dimethyl-2,3-dihydro-1*H*-inden-1-amine
[10] 6-(3,4-dimethyl-1-(2,2,2-trifluoroethyl)-1*H*-pyrazol-5-yl)-*N,N*-dimethyl-2,3-dihydro-1*H*-inden-1-amine

5. A process for preparing a compound of general formula (Ia) comprising: 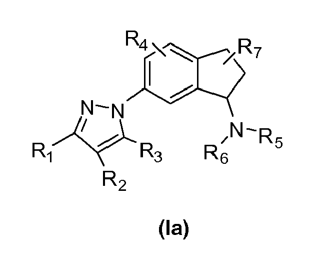
a) reacting a compound of general formula (II): 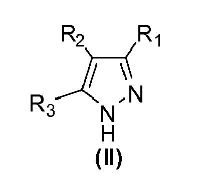 with a compound of general formula III: 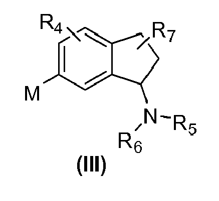 wherein M represents a halogen and R₁, R₂, R₃, R₄, R₅, R₆ and R₇ have the same meaning as in claims 1 or 2.

6. Process according to claim 5 wherein the reaction between compounds of general formula (II) and (III) is carried out in an organic solvent in the presence of a copper catalyst, a ligand and at least one base, the reaction being performed by subjecting the reaction mixture to reflux by conventional heating or by microwave radiation.

7. Process for preparing a compound of general formula (Ib) comprising: 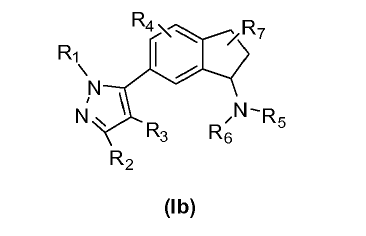
a) reacting a compound of general formula (VII): 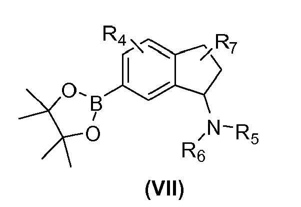 with a compound of general formula (VIII): 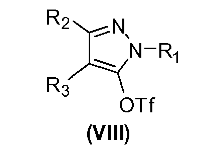 wherein M represents a halogen and R₁, R₂, R₃, R₄, R₅, R₆ and R₇ have the same meaning as in claims 1 or 2.

8. A process according to claim 7 wherein the reaction between compounds of general formula (VII) and (VIII) is carried out in an organic solvent in the presence of a copper catalyst, a ligand and at least one base, the reaction being performed by subjecting the reaction mixture to reflux by conventional heating or by microwave radiation.

9. Medicament comprising at least one compound according to any of claims 1 to 4.

10. Medicament comprising at least one compound according to any of claims 1 to 4 optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

11. Use of at least one compound according to any of claims 1 to 4 for the manufacture of a medicament for the treatment of 5-HT7 receptor mediated diseases or conditions.

12. Use of at least one compound according to any of claims 1 to 4 optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of 5-HT7 receptor mediated diseases or conditions.

13. Use according to any of claims 11 or 12 where the 5HT7 receptor mediated disease or condition is pain, preferably visceral pain, chronic pain, cancer pain, migraine, acute pain or neuropathic pain, more preferably neuropathic pain, allodynia or hyperalgesia.

14. Use according to any of claims 11 or 12 where the 5HT7 receptor mediated disease or condition is sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.
